# EUROPEAN PATENT SPECIFICATION

(11) **EP 2 654 688 B1**
(45) Date of publication and mention of the grant of the patent: **02.11.2016**
(21) Application number: 11793395.2
(22) Date of filing: 01.12.2011
(51) Int. Cl.: A61K 8/35, A61K 8/49, A61Q 17/04, A61K 8/368

(54) **A HIGH SPF SUNSCREEN COMPOSITION**
SONNENSCHUTZZUSAMMENSETZUNG MIT HOHEM LICHTSCHUTZFAKTOR
COMPOSITION D'ÉCRAN SOLAIRE À FACTEUR DE PROTECTION SOLAIRE ÉLEVÉ

(30) Priority: 20.12.2010 IN MU34432010; 28.02.2011 EP 11156135
(43) Date of publication of application: 30.10.2013
(73) Proprietor: Unilever N.V., 3013 AL Rotterdam (NL); Unilever PLC, London EC4Y 0DN (GB)
(72) Inventor: DUGGAL, Charu, 400 099 Mumbai (IN); GAURAV, Kumar, 825301 Jharkhand (IN); RAUT, Janhavi, Sanjay, MK44 1LQ Bedfordshire (GB)
(74) Representative: Corsten, Michael Allan
(86) International application number: PCT/EP2011/071454
(87) International publication number: WO 2012/084442

(56) References cited:
- WO-A1-2007/042259
- WO-A1-2008/022946
- WO-A2-02/055029
- WO-A2-02/058621
- US-A1- 2007 134 174

## Description

### Filed of the invention

The invention relates to a high Sun Protection Factor (SPF) solid sunscreen composition.

### Background of the invention

Solar radiation includes about 5% ultraviolet (UV) radiation, wavelength of which is between 200 nm and 400 nm. It is further classified into three regions: from 320 to 400 nm (UV-A), 290 to 320 nm (UV-B) and from 200 to 290 nm (UV-C). A large part of UV-C radiation is absorbed by the ozone layer, in the upper atmosphere. Exposure to UV-A and UV-B radiation for short periods of time causes reddening of the skin and localized irritation, whereas continued and prolonged exposure can lead to sunburn, melanoma and formation of wrinkles. UV radiation is also known to cause damage to hair. Therefore, it is desirable to protect the skin and other keratinous substrates of the human body from the harmful effects of both UV-A and UV-B radiation.

Various cosmetic preparations have been reported for preventing and/or protecting the skin from harmful effects of ultraviolet radiation. Numerous organic sunscreen agents capable of absorbing UV-A rays are reported in the field of cosmetics amongst which a particularly useful sunscreen is of the dibenzoylmethane class. Many UV-B sunscreens are also known and approved for safe use in personal care compositions for protection from UV-B radiation. Many cosmetic manufacturers prefer to include both UV-A and UV-B sunscreens in photoprotective compositions so as to provide protection over the entire range of UV radiation. Sun protection Factor (SPF) is a commonly measured attribute of photoprotective compositions which indicates the protection that the skin gets from exposure to both UV-B and UV-A radiation.

Thus, cosmetic manufacturers try to provide consumers with products having higher and higher SPF. One of the ways of achieving this is to incorporate high levels of UV-A and UV-B sunscreens. A disadvantage of incorporating high levels of sunscreens is the high cost of such sunscreens thus making the composition expensive. Further, there are safety and regulatory limitations on the upper limit of incorporation of these sunscreens. Sensory properties also reported to get affected on incorporation of high levels of sunscreens. Hence, there is a problem of achieving high SPF while keeping the total amount of sunscreens in the compositions relatively low.

Various publications on more effective sunscreen compositions have been reported. One such patent publication is US 6 224 852 which discloses liquid personal wash compositions comprising a sunscreen component and minimal required levels of cationic polymer. Additionally, nonionic surfactants can also be present in the composition alone or together with anionic or other surfactants to provide a cleansing and mildness effect. The compositions provide high deposition of sunscreen component; good SPF ("sun protection factor") values; and good consumer acceptable levels of lather.

US 6 048 517 discloses sunblocking compositions for application to the skin having SPF values higher than 40 and containing active sunscreening ingredients selected from the group consisting of homosalate, octyl salicylate and mixtures thereof, in combination with oxybenzone and optionally also containing octyl methoxycinnamate or avobenzone, or both.

Both of the above patent publications disclose compositions that comprise very high levels of organic sunscreens (higher than 8%) to achieve the high SPF. Therefore there exists a need for a personal care composition comprising sunscreen agents in low concentrations that are able to provide much higher SPF as compared to known sunscreen compositions comprising such low levels of sunscreen agents. It is desirable if the enhanced SPF benefit could be achieved through synergistic interaction of commonly used ingredients thereby giving the desired photoprotection benefits at substantially low costs.

The present applicants have been working on solving this problem and have surprisingly found that cosmetic compositions comprising dibenzoylmethane or its derivative in combination with an oil soluble UV-B sunscreen when incorporated in a sunscreen composition along with a non-ionic surfactant of a select class meeting certain HLB requirements provide the enhanced SPF benefits when applied on the substrate of interest.

It is therefore an object of the present invention to obviate at least some drawbacks of the prior art and provide high SPF (equal to or greater than 16) photo-protective sunscreen composition.

Another object of the present invention is to achieve the above object using relatively low amounts of sunscreen agents thereby keeping costs low.

Yet another object of the present invention is to provide high SPF sunscreen solid without compromising on the desired skin sensorial properties.

### Summary of the invention

According to one aspect of the present invention there is provided a solid sunscreen composition for obtaining an SPF higher than 16 according to claim 1.

According to another aspect of the present invention there is provided use of a composition of the first aspect of the invention for obtaining a SPF higher than about 16.

### Detailed description of the invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

By "A Sunscreen Composition" as used herein, is meant to include a composition for topical application to sun-exposed areas of the skin and/or hair of mammals, especially humans. Such a composition may be generally classified as leave-on or rinse off, and includes any product applied to a human body for also improving appearance, cleansing, odor control or general aesthetics. It is more preferably a leave-on product. The composition of the present invention can be in the form of a liquid, lotion, cream, foam, scrub, gel, or toner, or applied with an implement or via a face mask, pad or patch. Non-limiting examples of such sunscreen compositions include leave-on skin lotions, creams, antiperspirants, deodorants, lipsticks, foundations, mascara, sunless tanners and sunscreen lotions. "Skin" as used herein is meant to include skin on the face and body (e.g. neck, chest, back, arms, underarms, hands, legs, buttocks and scalp) and especially to the sun exposed parts thereof. The composition of the invention is also of relevance to applications on any other keratinous substrates of the human body other than skin e.g. hair where products may be formulated with specific aim of providing photoprotection.

By 'A High SPF sunscreen composition' is meant a composition that has an SPF higher than 16, more preferably higher than 18, further more preferably higher than 20. It is preferred that high SPF is achieved using total UV-B sunscreens in the range of 0.1 to 7%, preferably from 0.5 to 6%, more preferably 1 to 5% by weight of the composition. It is an advantage of the present invention that the high SPF values are achieved by using low amount of total organic sunscreens. By low amount of total organic sunscreens is meant that the total amount of organic sunscreens is less then 8%, preferably less than 7%, further more preferably less than 6% by weight of the composition.

The first aspect of the invention provides for a solid sunscreen composition. By a solid sunscreen composition is meant a composition that has a critical shear stress (apparent yield stress) as defined by H A Barnes (Handbook of Elementary Rheology, University of Wales Aberystwyth, 71-73 (2000)) of higher than 20 Pa, preferably higher than 50 Pa at 25°C. It is preferred that the solid composition has an apparent yield stress of less than 10 000 Pa at 25°C. It is preferred that the viscosity of solid compositions of the invention at critical shear stress is in the range of 1 000 to 500 000 Pa.s, preferably 1 000 to 100 000 Pa.s at 25°C. A preferred solid composition is a cream. Creams are usually known as soft solids. Thus, by the use of the term solid composition, in the present invention, are included soft-solid compositions.

Definitions of lotion and cream have been given by Brummer (Rheology Essentials of Cosmetic and Food Emulsions, Springer-Verlag Berline Heidelberg, 81-83 (2006)). Therein creams are defined as those compositions which do not flow out from a container at 25°C when it is turned upside down. Lotions are those compositions which flow out from the container at 25°C when turned upside down.

The invention provides for a high SPF sunscreen composition comprising a UV- A sunscreen which is a dibenzoylmethane or its derivative; an oil soluble UV-B organic sunscreen in low amount; selective amount of a selective non-ionic surfactant; and a cosmetically acceptable base.

The composition of the invention comprises 0.1 to 5% dibenzoylmethane or its derivative. Preferred dibenzoylmethane derivatives are selected from 4-tert-butyl-4'-methoxydibenzoylmethane, 2-methyldibenzoylmethane, 4-methyl-dibenzoyl-ethane, 4-isopropyldibenzoyl-methane, 4-tert-butyldibenzoylmethane, 2,4-dimethyldibenzoylmethane, 2,5-dimethyldibenzoylmethane, 4,4'-diisopropyl-dibenzoylmethane, 2-methyl-5-isopropyl-4'-methoxydibenzoylmethane, 2-methyl-5-tert-butyl-4'-methoxy-dibenzoyl methane, 2,4-dimethyl-4'-methoxy dibenzoylmethane or 2,6-dimethyl-4-tert-butyl-4'-methoxy-dibenzoylmethane. The most preferred dibenzoylmethane derivative is 4-tert.-butyl-4'-methoxydibenzoylmethane. Dibenzoylmethane or its derivative is preferably present in 0.2 to 5%, more preferably 0.4 to 3% by weight of the composition.

The composition of the invention comprises 0.1 to 7%, preferably from 0.5 to 6%, more preferably 1 to 5%, an oil soluble UV-B organic sunscreen by weight of the composition. The oil soluble UV-B organic sunscreen is preferably selected from the class of cinnamic acid, salicylic acid, diphenyl acrylic acid or derivatives thereof. A few of the preferred oil soluble UV-B sunscreens which are commercially available and useful for inclusion in the composition of the invention are Octisalate^{™}, Homosalate^{™}, NeoHelipan^{™}, Octocrylene^{™}, Oxybenzone^{™} or Parsol MCX^{™}. It is interesting to note that only use of oil soluble UV-B sunscreens in the composition of the present invention provide the enhanced SPF benefits of the invention while water-soluble UV-B sunscreens do not provide the desired benefits. It is preferred that the composition of the invention is substantially free of water soluble organic sunscreens. Water soluble sunscreens may however be incorporated in small amounts preferably less than 1%, further more preferably less than 0.5%, even further more preferably less than 0.1 % and optimally absent from the composition of the invention.

An important ingredient that contributes to enhancement of SPF of the sunscreen composition of the invention is a selective class of non-ionic surfactant. The non-ionic surfactant is selected from the class of (i) polyoxyethylene sorbitan alkyl esters with saturated C12 to C16 carbon chain and having HLB greater than 12 or (ii) polyoxyethylene sorbitan alkyl esters with unsaturated C18 carbon chain and having HLB greater than 9.

HLB is calculated using the Griffin method wherein HLB = 20 x Mh / M wherein Mh is the molecular mass of the hydrophilic portion of the molecule and M is the molecular mass of the whole molecule, giving a result on an arbitrary scale of 0 to 20. Typical values for various surfactants are given below:
- A value <10 : Lipid soluble (water insoluble)
- A value >10 : Water soluble
- A value from 4 to 8 indicates an anti-foaming atent
- A value from 7 to 11 indicates a W/O (water in oil) emulsifier
- A value from 12 to 16 indicates oil in water emulsion
- A value from 11 to 14 indicates a wetting agent
- A value from 12 to 15 is typical of detergents
A value of 16 to 20 indicates a solubiliser or hydrotrope

The above class of non-ionic surfactants have the general structure as given below: 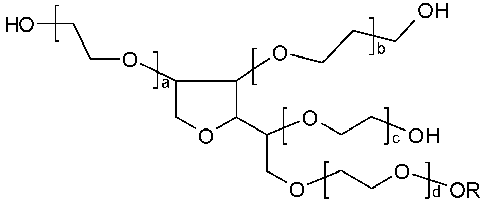 where R is long carbon chain.

It is observed that use of ionic surfactants or non-ionic surfactants of the polyoxyethylene sorbitan alkyl ester class not meeting the claimed criteria do not provide the desired SPF enhancement. Suitable examples of commercially available non-ionic surfactants for use in the composition of the invention are Tween 20, Tween 21, Tween 40, Tween 80, Tween 81 or Tween 85 trioleate. The non-ionic surfactant is included in 0.1 to 5%, preferably 0.2 to 3% by weight of the composition.

The structure of two suitable non-ionic surfactants, as per the invention is given below:

### Unsaturated - Oleic Chain

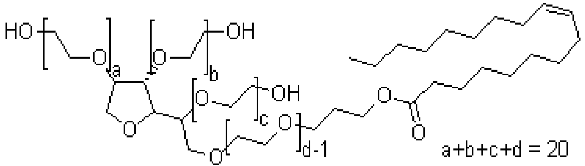

Tween 80
Polyoxyethylene sorbitan monooleate; sorbitan monooleate ethoxylate

### Saturated - Lauric Chain

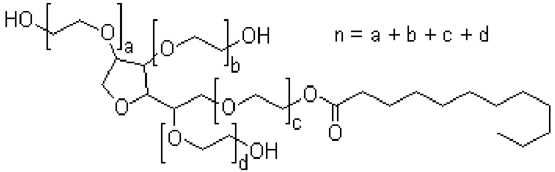

Tween 20
Polyoxyethylene sorbitan monolaurate

Other type of non-ionic surfactants which have similar structure but do not provide the desired benefits are those from the so called Span type (sorbitan alkyl esters). Typical example is given below.

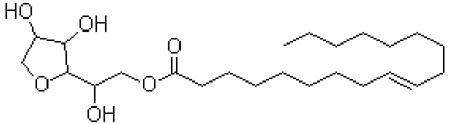

Span 20
Sorbitan laurate; sorbitan monododecanoate

In the solid composition of the invention a hydrophobically modified water soluble polymer is incorporated. These polymers are selected from hydrophobically modified polyacrylate (hydrophobically modified alkali swellable emulsion), modified celluloses preferably hydrophobically modified hydroxyethyl cellulose or ethyl hydoxyethyl cellulose.

Of these the preferred polymers are selected from the class of acrylate / R-methacrylate copolymer or crosspolymer, or an acrylate / R-alkyl acrylate crosspolymer; or copolymers of acryloyldimethyltaurate with methacrylate or R-alkyl acrylate, or hydrophobically modified cellulose.

Suitable polymers of the class of acrylate / R-methacrylate copolymer or crosspolymer, or an acrylate / R-alkyl acrylate crosspolymer, wherein R and alkyl are C₁₀₋₃₀ alkyl groups, include: (i) Acrylate/Beheneth-25 Methacrylate Copolymer, (ii) Acrylate/Steareth-20 Methacrylate Copolymer, (iii) Acrylate/Steareth-20 Methacrylate Crosspolymer, or (iv) Acrylates/C10-30 Alkyl Acrylate Crosspolymer.

R-methacrylate copolymer or crosspolymer has the general structure:

- (CH₂-CMeCO₂H)ₘ - (CH₂-CO₂R)ₙ -

R-alkyl acrylate crosspolymer has the general structure:

- (CH₂-CAlkylCO₂H)ₘ - (CH₂-CO₂R)ₙ-

wherein m and n are any whole integers in the range 500 - 10 000 000, preferably 500 - 1 000 000, most preferably 500 - 500 000.

Commercially available polymers of the class of polyacrylates useful for inclusion in the composition of the invention are Aculyn^{™} 22, Aculyn^{™} 28, or Aculyn^{™} 88, which are available from Rohm and Haas or Pemulen TR-2 which is available from Lubrizol. Also useful are Ucar Polyphobe 202/ 203/ 205 or 206.

Commercially available polymers of the class of copolymers of acryloyldimethyltaurate with methacrylate or R-alkyl acrylate include Aristoflex, Aristoflex HMB^{™}, Aristoflex BLV^{™}, Aristoflex AVS^{™} available from Clariant.

Commercailly available modified cellulose are Natrasols, Methocel class of products (e.g. Methocel 40-100, Methocel 40-101, Methocel 40-202).

When included, polymers from the above classes are preferably present in 0.2 % to 1.5% by weight of the composition.

Not wishing to be bound by theory, it is observed that inclusion of the specific non-ionic surfactant meeting the above criterion in the solid compositions of the invention provides the desired SPF enhancement, but adversely affects the sensory properties of the solid product when applied on the skin. It is only with inclusion of the polymers meeting the criterion as claimed, that the sensory properties are attained while maintaining the high SPF values. When polymers, not meeting the above criterion, are used, either the desired sensory properties are not achieved or the SPF enhancement is not attained

The composition of the invention comprises a cosmetically acceptable base. The cosmetically acceptable bases are such as to have a product in preferably a cream, lotion, gel or emulsion format. A more preferred solid form of the composition is a cream, further more preferably a vanishing cream. Vanishing cream base is one which comprises 3 to 25%, more preferably 5 to 20% fatty acid. The base preferably comprises 0.1 to 10%, more preferably 0.1 to 3% soap. C₁₂ to C₂₀ fatty acids are especially preferred in vanishing cream bases, further more preferred being C₁₄ to C₁₈ fatty acids. In creams, the fatty acid is preferably substantially a mixture of stearic acid and palmitic acid. Soaps in the vanishing cream base include alkali metal salt of fatty acids, like sodium or potassium salts The soap is preferably the potassium salt of the fatty acid mixture. The fatty acid in vanishing cream base is often prepared using hystric acid which is substantially (generally about 90 to 95%) a mixture of stearic acid and palmitic acid. Thus, inclusion of hysteric acid and its soap to prepare the vanishing cream base is within the scope of the present invention. It is particularly preferred that the composition comprises at least 6%, preferably at least 10%, more preferably at least 12% fatty acid. The cosmetically acceptable base is usually from 10 to 99.9%, preferably from 50 to 99% by weight of the composition. It has been observed that use of such high levels of fatty acid also contributes to the high SPF. The cosmetically acceptable base preferably includes water. Water is preferably included in 35 to 90%, more preferably 50 to 85%, further more preferably 50 to 80% by weight of the composition.

Other useful sun-protective agents e.g. inorganic sun-blocks may be preferably used in the present invention. These include, for example, zinc oxide iron oxide, silica, such as fumed silica, or titanium dioxide. The total amount of sun block that is preferably incorporated in the composition according to the invention is from 0.1 to 5% by weight of the composition.

The composition of the invention may additionally comprise a skin lightening agent. The skin lightening agent is preferably chosen from a vitamin B3 compound or its derivative e.g. niacin, nicotinic acid, niacinamide or other well known skin lightening agents e.g. aloe extract, ammonium lactate, azelaic acid, kojic acid, citrate esters, ellagic acid, glycolic acid, green tea extract, hydroquinone, lemon extract, linoleic acid, magnesium ascorbyl phosphate, vitamins like vitamin B6, vitamin B12, vitamin C, vitamin A, a dicarboxylic acid, resorcinol derivatives, hydroxycarboxylic acid like lactic acid and their salts e.g. sodium lactate, and mixtures thereof. Vitamin B3 compound or its derivative e.g. niacin, nicotinic acid, niacinamide are the more preferred skin lightening agent as per the invention, most preferred being niacinamide. Niacinamide, when used, is preferably present in an amount in the range of 0.1 to 10%, more preferably 0.2 to 5% by weight of the composition.

The composition according to the invention may also comprise other diluents. The diluents act as a dispersant or carrier for other materials present in the composition, so as to facilitate their distribution when the composition is applied to the skin. Diluents other than water can include liquid or solid emollients, solvents, humectants, thickeners and powders.

The composition of the invention may comprise a conventional deodourant base as the cosmetically acceptable carrier. By a deodorant is meant a product in the stick, roll-on, or propellant medium which is used for personal deodorant benefit e.g. application in the under-arm or any other area which may or may not contain antiperspirant actives.

Deodorant compositions can generally be in the form of firm solids, soft solids, gels, creams, and liquids and are dispensed using applicators appropriate to the physical characteristics of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

According to another aspect of the present invention there is provided use of a composition of the first aspect of the invention for obtaining SPF higher than16. The SPF is preferably higher than 18 further more preferably higher than 20. The use is preferably for non-therapeutic benefits.

The invention is now further described by way of the following non-limiting examples.

### Examples

### Examples 1 to 15: Effect of various non-ionic surfactants

Various compositions as shown in table 1 were prepared using different non-ionic surfactants. The difference between the various compositions was in the type of non-ionic surfactant used which are detailed in table 2. The SPF of the various compositions (examples 1-15) was measured and the results are shown in table 2. In-vitro SPF was measured using the Optometrics 290S instrument model. The substrate used was a 10 cm Transpore tape procured from 3M Company. The sample was applied at 2 mg/cm².

**Table 1**

| Ingredients | wt% |
|---|---|
| Stearic Acid | 15 |
| Parsol MCX^{™} | 3 |
| Parsol 1789^{™} | 1.5 |
| Non-ionic surfactant | 2 |
| Polymer, Carbomer 980 | 1 |
| Niacinamide | 1 |
| Glycerine | 1 |
| Isopropyl myristate | 1 |
| Titanium dioxide | 1 |
| Glyceryl stearate | 1 |
| Mineral oil | 1 |
| Tri ethanol amine | 0.5 |
| Potassium hydroxide | 0.5 |
| Cetyl alcohol | 1 |
| DC200 350 | 1 |
| Perfume | 0.5 |
| Methyl paraben + propyl paraben | 0.5 |
| Water | To 100 |

**Table 2**

| **Ex #** | **Surfactants** | **Formula** | **HLB** | **SPF** |
|---|---|---|---|---|
| 1 | Span 85 | C18:1*3EO1 | 1.8 | 7.9 |
| 2 | Span 65 | C18*3EO1 | 2.1 | 9.0 |
| 3 | Span 80 | C18:1EO1 | 4.3 | 15.2 |
| 4 | Span 60 | C18EO1 | 4.7 | 10.0 |
| 5 | Span 40 | C16EO1 | 6.0 | 11.0 |
| 6 | Span 20 | C12EO1 | 9.0 | 11.9 |
| 7 | Tween 61 | C18EO4 | 9.6 | 9.0 |
| 8 | Tween 60 | C18EO20 | 14.9 | 10.6 |
| 9 | Tween 65 Tristearate | C18*3EO20 | 10.5 | 9.0 |
| 10 | Tween 81 | C18:1EO5 | 10.0 | 19.0 |
| 11 | Tween 85, Trioleate | C18:1*3EO20 | 11.0 | 18.0 |
| 12 | Tween 21 | C12EO4 | 13.1 | 21.0 |
| 13 | Tween 80 | C18:1EO20 | 15.0 | 21.2 |
| 14 | Tween 40 | C16EO20 | 15.6 | 17.7 |
| 15 | Tween 20 | C12EO20 | 16.9 | 28.0 |

In table 2, in the formula
Cx refers to an alkyl chain with x carbon atoms
Cx:1 refers to an alkyl chain with x carbon atoms with one unsaturated bond in the carbon chain.
Cx*3 or Cx:1*3 refers to three alkyl chains
EOy refers to y ethylene oxide groups

The data in table 2 indicates that compositions outside the invention (examples 1 to 9) do not provide the desired high SPF while compositions within the invention (examples 10 to 15) provide high SPF values.

### Examples 16 to 19: Effect of inclusion of the polymer as per present invention in cream compositions

Cream compositions as shown in table 3 were prepared. The SPF of the compositions of examples 16 to 19 were measured as per procedure used for the previous examples and the SPF values were found to be comparable to example 15 i.e about 28. The viscosity of the compositions were measured using an AR-1000 model stress controlled Rheometer having a cone and plate geometry (cone: 40 mm diameter, 2 deg, truncation: 58 micron) over a shear rate ranging from 0.1 to 1000 s⁻¹ at a temperature of 25 °C. Each measurement over the above shear rate range was made over a time period of five minutes. Data on the viscosity of the samples at a representative shear rate of 1 s⁻¹ is shown in table 3..

**Table 3**

| Ingredients | Example - 16, wt% | Example - 17, wt% | Example - 18, wt% | Example - 19, wt% |
|---|---|---|---|---|
| Hystric Acid | 17.00 | 17.00 | 17.00 | 17.00 |
| Parsol MCX^{™} | 2.25 | 2.25 | 2.25 | 2.25 |
| Parsol 1789^{™} | 1.20 | 1.20 | 1.20 | 1.20 |
| Non-ionic surfactant Tween -20 | 2.00 | 2.00 | 2.00 | 2.00 |
| Polymer | - | Aculyn 88 1% | Aristoflex HMB 0.35% | Aculyn 28 1.0% |
| Niacinamide | 1.00 | 1.00 | 1.00 | 1.00 |
| Glycerine | 1.00 | 1.00 | 1.00 | 1.00 |
| Titanium dioxide | 1.00 | 1.00 | 1.00 | 1.00 |
| Potassium hydroxide | 0.57 | 0.57 | 0.57 | 0.57 |
| Silicone oil | 1.00 | 1.00 | 1.00 | 1.00 |
| Water | To 100 | To 100 | To 100 | To 100 |
| Viscosity (Pa. S) | 6.5 | 65 | 45 | 50 |

Data in table 3 indicates that for samples as per the invention where the claimed polymers were used (example 17 to 19) the desired high viscosity of a cream composition is obtained as compared to not using the desired polymer (example 16).

### Example 20 (not part of the invention)

A lotion composition as shown in table 4 was prepared.

**Table 4**

| Ingredients | wt% |
|---|---|
| Stearic Acid | 6.0 |
| Parsol MCX^{™} | 4.0 |
| Parsol 1789^{™} | 2.0 |
| Non-ionic surfactant, Tween -20 | 3.0 |
| Niacinamide | 3.0 |
| Glycerine | 1.0 |
| Isopropyl myristate | 3.0 |
| Titanium dioxide | 0.2 |
| Glycerol mono stearate | 1.5 |
| Mineral oil | 1.5 |
| Tri ethanol amine | 0.7 |
| DC200 | 0.5 |
| Methyl paraben + Propyl paraben | 0.3 |
| Water | To 100 |

The composition as prepared in table 4 provided a high SPF value of 24 measured as per procedure used for the previous examples.

The invention thus provides for a high SPF solid sunscreen composition comprising relatively low amount of sunscreen compounds and this is achieved using well known low cost materials thereby achieving the overall benefits at low cost.

## Claims

1. A solid sunscreen composition for obtaining an SPF higher than 16 comprising:
a) less than 8% by weight total organic sunscreens comprising,
b) 0.1 % to 5 % by weight dibenzoylmethane or its derivative;
c) 0.1 to 7 % by weight an oil soluble UV-B organic sunscreen;
d) 0.1 to 5% by weight non-ionic surfactant selected from the class of polyoxyethylene sorbitan alkyl esters with saturated C12 to C16 carbon chain and having HLB greater than 12 or from the class of polyoxyethylene sorbitan alkyl esters with unsaturated C18 carbon chain and having HLB greater than 9;
e) 0.1 to 2.0 % w/w of a hydrophobically modified water soluble polymer selected from the class of polyacrylates or modified cellulose; and
f) a cosmetically acceptable base, wherein said cosmetically acceptable base is a cream comprising 3 to 25% fatty acid.

2. A composition as claimed in any one of the preceding claims comprising 0.1 to 10% soap.

3. A composition as claimed in any one of the preceding claims wherein said non-ionic surfactant has the chemical formula C12EO20, C12EO4 or C18:1EO20.

4. A composition as claimed in any one of the preceding claims wherein said oil soluble UV-B organic sunscreen is selected from the group consisting of cinnamic acid, salicylic acid, diphenyl acrylic acid and derivatives thereof.

5. A composition as claimed in any one of the preceding claims wherein said dibenzoyl methane derivative is 4-tert.-butyl-4'-methoxydibenzoylmethane.

6. Composition as claimed in any one of the preceding claims for r use in a method of obtaining SPF higher than 16.

## Patentansprüche

1. Feste Sonnenschutzzusammensetzung zur Erzielung eines SPF von höher als 16, umfassend:
a) weniger als 8 Gewichts-% gesamte organische Sonnenschutzmittel, umfassend
b) 0,1 bis 5 Gewichts-% Dibenzoylmethan oder sein Derivat,
c) 0,1 bis 7 Gewichts-% eines organischen öllöslichen UV-B-Sonnenschutzmittels,
d) 0,1 bis 5 Gewichts-% eines nicht-ionischen Tensids, ausgewählt aus der Klasse von Polyoxyethylensorbitanalkylestern mit gesättigter C₁₂- bis C₁₆-Kohlenstoffkette und mit einem HLB von mehr als 12 oder aus der Klasse von Polyoxyethylensorbitanalkylestern mit ungesättigter C₁₈-Kohlenstoffkette und mit einem HLB von mehr als 9,
e) 0,1 bis 2,0% Gew./Gew. eines hydrophob modifizierten wasserlöslichen Polymers, ausgewählt aus der Klasse von Polyacrylaten oder modifizierter Cellulose, und
f) einen kosmetisch annehmbaren Grundbestandteil, wobei der kosmetisch annehmbare Grundbestandteil eine Creme darstellt, die 3 bis 25% Fettsäure umfasst.

2. Zusammensetzung, wie in irgendeinem der vorhergehenden Ansprüche beansprucht, die 0,1 bis 10% Seife umfasst.

3. Zusammensetzung, wie in irgendeinem der vorhergehenden Ansprüche beansprucht, wobei das nicht-ionische Tensid die chemische Formel C12EO20, C12EO4 oder C18:1EO20 aufweist.

4. Zusammensetzung, wie in irgendeinem der vorhergehenden Ansprüche beansprucht, wobei das öllösliche organische UV-B-Sonnenschutzmittel aus der Gruppe ausgewählt ist, die aus Zimtsäure, Salicylsäure, Diphenylacrylsäure und Derivaten davon besteht.

5. Zusammensetzung, wie in irgendeinem der vorhergehenden Ansprüche beansprucht, wobei das Dibenzoylmethan-Derivat 4-tert.-Butyl-4'-methoxydibenzoylmethan darstellt.

6. Zusammensetzung, wie in irgendeinem der vorhergehenden Ansprüche beansprucht, zur Verwendung in einem Verfahren zum Erzielen eines SPF von mehr als 16.

## Revendications

1. Composition d'écran solaire solide pour obtenir un SPF supérieur à 16 comprenant :
a) moins de 8 % en masse d'écrans solaires organiques totaux comprenant,
b) de 0,1 % à 5 % en masse de dibenzoylméthane ou son dérivé
c) de 0,1 à 7 % en masse d'un écran solaire organique UV-B soluble dans l'huile ;
d) de 0,1 à 5 % en masse de tensioactif non ionique choisi parmi la classe des esters alkyliques de polyoxyéthylène sorbitane avec une chaîne carbonée en C12 à C16 saturée et ayant un HLB supérieur à 12 ou parmi la classe des esters alkyliques de polyoxyéthylène sorbitane avec une chaîne carbonée en C18 insaturée et ayant un HLB supérieur à 9 ;
e) de 0,1 à 2,0 % en masse/masse d'un polymère soluble dans l'eau hydrophobiquement modifié choisi parmi la classe des polyacrylates ou de la cellulose modifiée ; et
f) une base cosmétiquement acceptable, dans laquelle ladite base cosmétiquement acceptable est une crème comprenant de 3 à 25 % d'acide gras.

2. Composition selon l'une quelconque des revendications précédentes comprenant de 0,1 à 10 % de savon.

3. Composition selon l'une quelconque des revendications précédentes, dans laquelle ledit tensioactif non ionique présente la formule chimique C12EO20, C12EO4 ou C18:1EO20.

4. Composition selon l'une quelconque des revendications précédentes, dans laquelle ledit écran solaire organique UV-B soluble dans l'huile est choisi dans le groupe constitué d'acide cinnamique, d'acide salicylique, d'acide diphénylacrylique et de dérivés de ceux-ci.

5. Composition selon l'une quelconque des revendications précédentes, dans laquelle ledit dérivé de dibenzoylméthane est le 4-tert.-butyl-4'-méthoxydibenzoylméthane.

6. Composition selon l'une quelconque des revendications précédentes pour une utilisation dans un procédé d'obtention de SPF supérieur à 16.
